# EUROPEAN PATENT APPLICATION

(11) **EP 4 520 332 A1**
(43) Date of publication of application: **12.03.2025**
(21) Application number: 23799719.2
(22) Date of filing: 04.05.2023
(51) Int. Cl.: A61K 31/4745, A61K 31/4741, A61P 25/28, A23L 33/10

(54) **COMPOSITION FOR PROMOTING MITOPHAGY ACTIVITY COMPRISING ISOQUINOLINE DERIVATIVE COMPOUND AS ACTIVE INGREDIENT AND USE THEREOF**

(30) Priority: 06.05.2022 KR 20220056058
(71) Applicant: Altmedical Co., Ltd., Seoul 02792 (KR); Dong-A University Research Foundation for Industry-Academy Cooperation, Busan 49315 (KR)
(72) Inventor: YUN, Jeanho, Busan 47863 (KR); CHO, Jong Hyun, Gimhae-si, Gyeongsangnam-do 51003 (KR); UM, Jee-Hyun, Busan 49446 (KR); SHIN, Dong Jin, Busan 46512 (KR); KIM, Young Yeon, Busan 49202 (KR); CHOI, Semyeong, Busan 47581 (KR)
(74) Representative: Nederlandsch Octrooibureau
(86) International application number: PCT/KR2023/006153
(87) International publication number: WO 2023/214838

(57) **Abstract**

The present disclosure relates to novel isoquinoline derivatives. The derivatives have an effect of promoting mitophagy activity, thereby reducing mitochondria with structural and/or functional damage. In particular, the novel isoquinoline derivatives according to the present disclosure do not induce mitochondrial dysfunction and have been identified as safe compounds that are non-toxic to cells, and thus can be utilized for the prevention, amelioration, and/or treatment of various diseases that may be caused by abnormal mitochondria.

## Description

### [Technical Field]

The present disclosure relates to a composition for promoting mitophagy activity comprising an isoquinoline derivative compound as an active ingredient, and to uses thereof.

The present disclosure claims priority based on Korean Patent Application No. 10-2022-0056058, filed on May 6, 2022, and all contents disclosed in the specifications and drawings of these applications are incorporated herein by reference.

### [Background Art]

Mitochondria are generally organelles present within cells, commonly known as the "powerhouses of the cell." Mitochondria not only produce energy through oxidative phosphorylation in cells but also perform essential functions for the survival of cells and organisms, including apoptosis, ion homeostasis, intermediary metabolism of sugars and lipids, pyrimidine synthesis, urea synthesis, and heme synthesis.

The activity of mitochondria in vivo is maintained by a balance of mitochondrial production, fusion, and fission mechanisms, as well as the production of new mitochondria and the removal of damaged or expired mitochondria through mitophagy activity. Reactive oxygen species generated from damaged mitochondria due to abnormalities in mitophagy activity have been considered a major cause of aging, and the accumulation of damaged mitochondria is be closely associated with various metabolic disease, including diabetes, as well as aging-related degenerative disease (Bratic and Larsson, 2013).

Mitophagy is an intracellular degradation mechanism that removes damaged or unnecessary mitochondria. When mitochondrial damage occurs, it forms an autophagosome by surrounding the damaged mitochondria with a membrane, which then fuses with a lysosome to selectively eliminate the damaged mitochondria. The activity of mitophagy is be important in regulating mitochondrial function in various cells, including neurons, and in maintaining tissue functionality.

Mitophagy is a selective degradation mechanism for mitochondria. Initially, it was considered part of autophagy, a process that breaks down various intracellular components to survive under nutrient-deprived conditions. However, it was later identified as a mechanism capable of effectively eliminating damaged or unnecessary mitochondria by surrounding them with a membrane, fusing with a lysosome, and completely degrading them. Mitophagy plays a role in protecting mitochondria by preventing the release of pro-apoptotic proteins, the generation of reactive oxygen species, and the unnecessary hydrolysis of ATP by aged, damaged, and depolarized mitochondria.

According to recent research, inhibition of mitophagy activity can lead to the accumulation of damaged mitochondria, inducing the death of motor neurons and potentially causing degenerative brain disease like Parkinson's disease. Additionally, abnormalities in mitophagy activity have been reported to be related to a wide range of human disease, including degenerative brain disease such as Parkinson's, Alzheimer's, and Lou Gehrig's disease, as well as peripheral neuropathy, heart disease, metabolic disease, and cancer, heightening researchers' interest in the role of mitophagy in human disease and its potential therapeutic applications.

Currently, experimental methods to induce mitophagy activity involve treating with so-called 'mitochondrial toxins', such as CCCP, FCCP, and rotenone, which induce mitochondrial dysfunction. However, the CCCP and FCCP are mitochondrial membrane potential uncouplers that depolarize the mitochondrial membrane potential, and rotenone acts as a Complex I inhibitor. These mitochondrial toxins induce mitophagy activity, a mechanism for removing damaged mitochondria, by directly causing mitochondrial damage, but their strong toxicity to cells limits their use as drugs to promote mitophagy activity.

### [Disclosure]

### [Technical Problem]

The object of the present disclosure is to provide a pharmaceutical composition for promoting mitophagy activity, comprising an isoquinoline derivative compound or a salt thereof as an active ingredient.

Another object of the present disclosure is to provide a food composition for promoting mitophagy activity, comprising an isoquinoline derivative compound or a salt thereof as an active ingredient.

Another object of the present disclosure is to provide a pharmaceutical composition for the prevention or treatment of disease caused by mitochondrial dysfunction, comprising an isoquinoline derivative or a salt thereof as an active ingredient. Another object of the present disclosure is to provide a food composition for the prevention or improvement of disease caused by mitochondrial dysfunction, comprising an isoquinoline derivative or a salt thereof as an active ingredient. Another object of the present disclosure is to provide a method for increasing mitophagy activity in cells, comprising the step of treating cells with an isoquinoline derivative compound in vitro.

Another object of the present disclosure is to provide a method for inhibiting mitochondrial dysfunction, comprising the step of treating cells with an isoquinoline derivative compound in vitro.

### [Technical Solution]

To achieve the above object, the present disclosure provides a pharmaceutical composition for promoting mitophagy activity, comprising an isoquinoline derivative compound or a salt thereof as an active ingredient.

Additionally, the present disclosure provides a food composition for promoting mitophagy activity, comprising an isoquinoline derivative compound or a salt thereof as an active ingredient.

Furthermore, the present disclosure provides a use of the isoquinoline derivative compound or a salt thereof for the preparation of a medicament for promoting mitophagy activity.

Furthermore, the present disclosure provides a method for promoting mitophagy activity, comprising the step of administering the isoquinoline derivative compound or a salt thereof to an individual in need thereof.

Additionally, the present disclosure provides a pharmaceutical composition for the prevention or treatment of disease caused by mitochondrial dysfunction, comprising an isoquinoline derivative or a salt thereof as an active ingredient.

Additionally, the present disclosure provides a food composition for the prevention or improvement of disease caused by mitochondrial dysfunction, comprising an isoquinoline derivative or a salt thereof as an active ingredient.

Furthermore, the present disclosure provides a use of an isoquinoline derivative compound or a salt thereof for the preparation of a medicament for the prevention, improvement, and/or treatment of disease caused by mitochondrial dysfunction. Furthermore, the present disclosure provides a method for the prevention or treatment of disease caused by mitochondrial dysfunction, comprising the step of administering the isoquinoline derivative compound or a salt thereof to an individual in need thereof. Furthermore, the present disclosure provides a use of an isoquinoline derivative compound or a salt thereof for the prevention, improvement, and/or treatment of disease caused by mitochondrial dysfunction.

Additionally, the present disclosure provides a method for increasing mitophagy activity in cells, comprising the step of treating cells with an isoquinoline derivative compound in vitro.

Additionally, the present disclosure provides a method for inhibiting mitochondrial dysfunction, comprising the step of treating cells with an isoquinoline derivative compound in vitro.

Furthermore, the present disclosure provides a use of an isoquinoline derivative compound or a salt thereof for the preparation of a medicament for the improvement or inhibition of mitochondrial dysfunction.

Furthermore, the present disclosure provides a method for the improvement or inhibition of mitochondrial dysfunction, comprising the step of administering the isoquinoline derivative compound or a salt thereof to an individual in need thereof. Furthermore, the present disclosure provides a use of an isoquinoline derivative compound or a salt thereof for the improvement or inhibition of mitochondrial dysfunction.

In one exemplary embodiment of the present disclosure, the isoquinoline derivative compound may be represented by the following Chemical Formula 1 to Chemical Formula 6, but is not limited thereto:

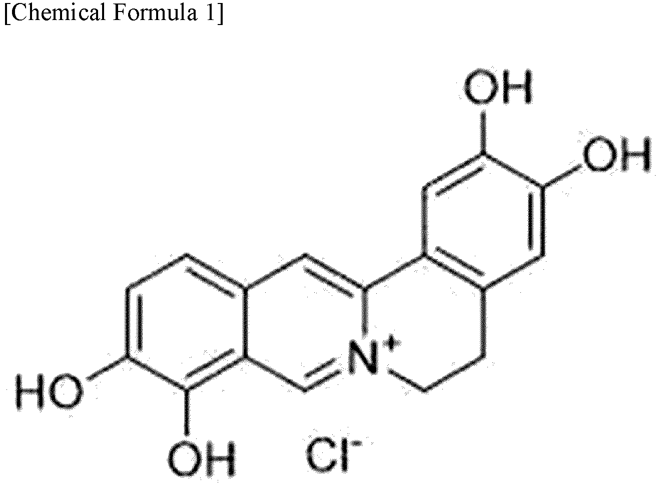

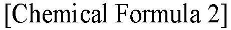

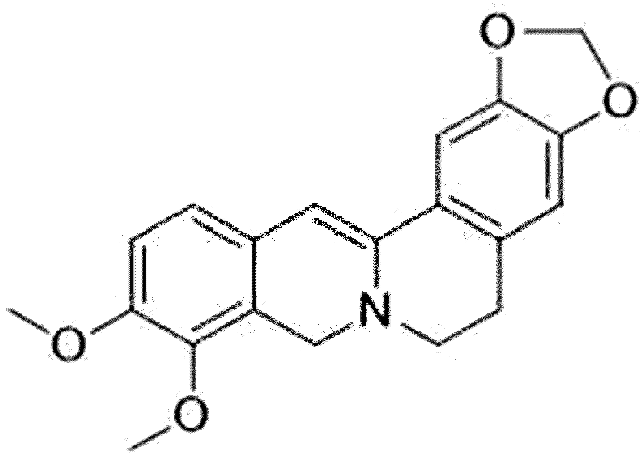

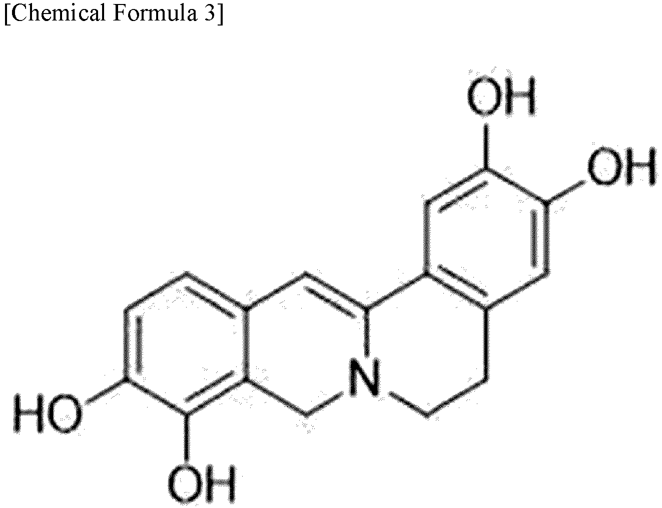

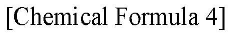

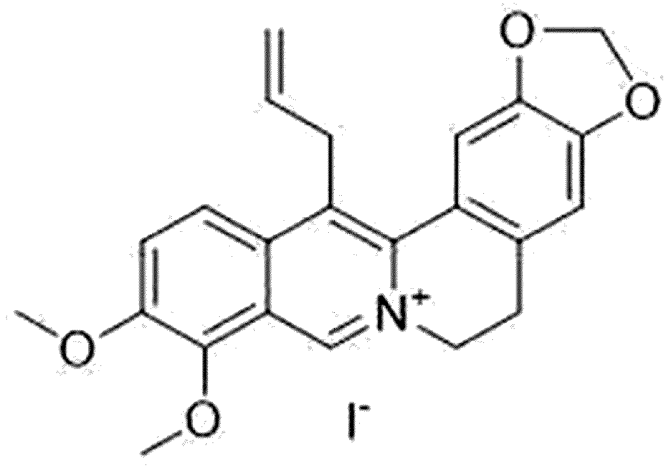

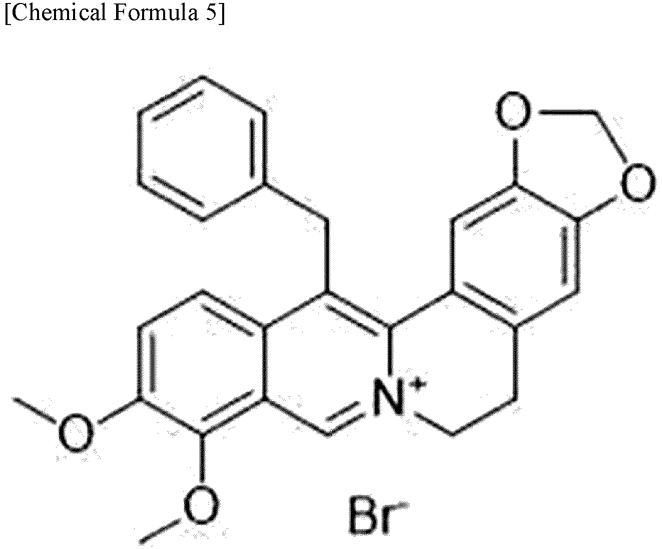

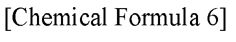

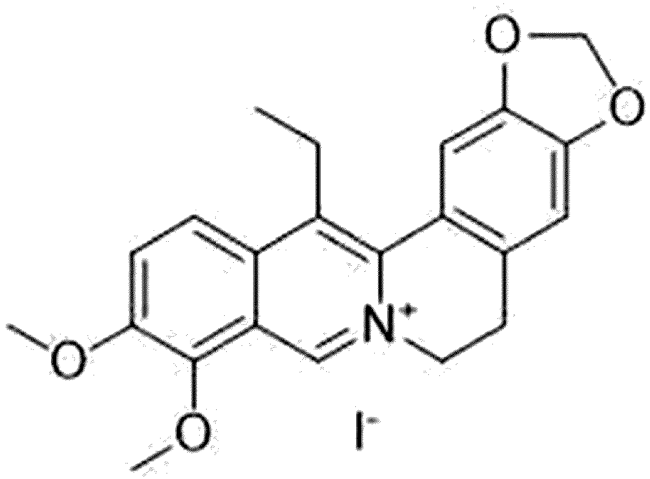

In another exemplary embodiment of the present disclosure, the isoquinoline derivative compound may be capable of increasing mitophagy activity, but is not limited thereto.

In yet another exemplary embodiment of the present disclosure, the increase in mitophagy activity may involve the removal of dysfunctional mitochondria, but is not limited thereto. That is, the increase in mitophagy activity may refer to an increase in the removal of dysfunctional mitochondria or a decrease in dysfunctional mitochondria.

In yet another exemplary embodiment of the present disclosure, the dysfunctional mitochondria may be capable of inducing neurodegenerative disease, but is not limited thereto.

In yet another exemplary embodiment of the present disclosure, the disease caused by mitochondrial dysfunction may be a neurodegenerative disease, but is not limited thereto.

In yet another exemplary embodiment of the present disclosure, the neurodegenerative disease may be selected from the group consisting of Alzheimer's disease, Parkinson's disease, amyotrophic lateral sclerosis, Huntington's disease, multiple sclerosis, immune-mediated brain dysfunction, progressive neurodegenerative disease, metabolic brain disease, Niemann-Pick disease, cerebral ischemia, and dementia due to cerebral hemorrhage, but are not limited thereto.

In yet another exemplary embodiment of the present disclosure, the neurodegenerative disease may be Alzheimer's disease or dementia, but are not limited thereto.

### [Advantageous Effects]

The present disclosure relates to novel isoquinoline derivatives. The derivatives have an effect of promoting mitophagy activity, thereby reducing mitochondria with structural and/or functional damage. In particular, the novel isoquinoline derivatives according to the present disclosure do not induce mitochondrial dysfunction and have been identified as safe compounds that are non-toxic to cells, and thus can be utilized for the prevention, amelioration, and/or treatment of various diseases that may be caused by abnormal mitochondria.

### [Brief Description of the Drawings]

Figure 1 is a diagram confirming the structure of the isoquinoline derivatives of the present disclosure.
Figure 2 is a diagram confirming the mitophagy activity promoting effect of the isoquinoline derivatives of the present disclosure, verified through flow cytometry (A: quantification of mitophagy activity for six types of derivatives, B: quantification of mitophagy activity according to CD1-386 and CD1-409 concentrations).
Figure 3 is a diagram analyzingthe effect of the isoquinoline derivatives of the present disclosure on mitochondrial membrane potential using a TMRM assay.
Figure 4 is a diagram confirming the cytotoxicity of the isoquinoline derivatives of the present disclosure using a colony forming assay.

### [Mode of the Invention]

The exemplary embodiments of the present disclosure will now be described in detail with reference to the accompanying drawings. In the following description, a detailed explanation of techniques well-known to those skilled in the art may be omitted. Additionally, in describing the present disclosure, specific details of related known functions or configurations may be omitted if it is determined that a detailed explanation could unnecessarily obscure the gist of the present disclosure. Additionally, the terminology used in this specification is chosen to appropriately describe the preferred exemplary embodiments of the present disclosure and may vary depending on the intentions of the user, operator, or customary practices in the relevant field of the present disclosure.

Therefore, the definitions of these terms should be based on the entirety of the present specification. Throughout the specification, when a part is described as "including" a certain component, it means that, unless otherwise specified, the inclusion of other components is not excluded and that additional components may be included.

The present disclosure provides a pharmaceutical composition for promoting mitophagy activity, comprising an isoquinoline derivative compound or a salt thereof as an active ingredient.

Throughout the present specification, the term "isoquinoline derivative" may be used interchangeably with "isoquinoline derivative compound."

Throughout the present specification, the term "compound" refers to a concept that includes both the isoquinoline derivative compound and a salt thereof.

**In** the present disclosure, the derivative includes pharmaceutically acceptable salts, pharmaceutically acceptable solvates, pharmaceutically acceptable hydrates, pharmaceutically acceptable anhydrates, pharmaceutically acceptable enantiomers, pharmaceutically acceptable esters, pharmaceutically acceptable polymorphs, pharmaceutically acceptable prodrugs, pharmaceutically acceptable complexes, and the like.

In the present disclosure, the term "salt" includes both pharmaceutically acceptable salts and food-grade acceptable salts. The pharmaceutically or food-grade acceptable salt refers to a salt derived from a pharmaceutically or food-grade acceptable organic acid, inorganic acid, or base.

Examples of suitable acids include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, gluconic acid, naphthalene-2-sulfonic acid, benzenesulfonic acid, and etc. The acid addition salt can be prepared by conventional methods, for example, by dissolving the compound in an excess aqueous solution of the acid and precipitating the salt using a polar organic solvent such as methanol, ethanol, acetone, or acetonitrile. Additionally, the salt can be prepared by heating an equimolar amount of the compound and an acid or alcohol in water, followed by evaporating the mixture to dryness, or by isolating the precipitated salt through suction filtration.

Salts derived from suitable bases may include alkali metals such as sodium and potassium, alkaline earth metals such as magnesium, and ammonium, but is not limited thereto. Alkali metal or alkaline earth metal salts can be obtained by, for example, dissolving the compound in an excess solution of alkali metal hydroxide or alkaline earth metal hydroxide, filtering out the insoluble compound salt, and then evaporating and drying the supernatant. In this case, it is particularly suitable from a pharmaceutical standpoint to prepare metal salts, specifically sodium, potassium, or calcium salts. Additionally, the corresponding silver salts can be obtained by reacting the alkali metal or alkaline earth metal salts with a suitable silver salt (e.g., silver nitrate).

The scope of the compounds of the present disclosure includes not only pharmaceutically acceptable salts but also all isomers, hydrates, and solvates that can be produced by conventional methods.

The term "prevention" as used in the present disclosure refers to any act of suppressing or delaying the symptoms of a specific disease through the administration of the composition of the present disclosure.

The term "treatment" as used in the present disclosure refers to any act of improving or beneficially modifying the symptoms of a specific disease through the administration of the composition of the present disclosure.

The term "mitophagy" as used in the present disclosure refers to an intracellular degradation mechanism that removes damaged or unnecessary mitochondria. When mitochondrial damage occurs, it can form an autophagosome, which fuses with lysosomes to selectively degrade and remove the damaged mitochondria. Mitophagy is distinguished from autophagy, which is a mechanism that generates macromolecular precursors and produces energy by degrading and recycling unnecessary cellular components (such as old proteins, protein aggregates, organelles, and pathogens that have infiltrated the cell) when the cell is in a state of nutrient deficiency, among other conditions. Mitophagy is regulated independently of the regulatory signals such as nutrients, energy, and stress that control autophagy. In the present disclosure, the phrase "promotes mitophagy" encompasses all actions that enhance or increase the activity, frequency, extent, and level of mitophagy.

The composition according to the present disclosure may additionally include a pharmaceutically acceptable carrier. The term "pharmaceutically acceptable" in the present disclosure refers to a characteristic indicating that there is no toxicity to the cells or humans exposed to the composition. The carrier may be any of those known in the art, including but not limited to buffers, preservatives, anesthetics, solubilizers, emulsifiers, stabilizers, excipients, and lubricants.

Additionally, the composition of the present disclosure can be formulated for use in various forms, including oral formulations such as powders, granules, tablets, capsules, suspensions, emulsions, syrups, and aerosols, as well as topical preparations, suppositories, and sterile injectable solutions, according to conventional methods. Furthermore, it can be used in the form of topical skin preparations such as ointments, lotions, sprays, patches, creams, powders, suspensions, gels, or jellies. Excipients, carriers, and diluents that may be included in the composition of the present disclosure include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil, among others. When formulating, the composition is prepared using commonly used diluents or excipients such as fillers, bulking agents, binders, wetting agents, disintegrants, and surfactants.

Solid formulations for oral administration include tablets, powders, granules, and capsules, and these solid forms are prepared by mixing one or more excipient with the extract of Psychotria rubra, such as starch, calcium carbonate, sucrose, lactose, or gelatin, among others. Additionally, lubricants such as magnesium stearate and talc are also used. Liquid formulations for oral use include suspensions, solutions, emulsions, and syrups, and may contain various excipients in addition to commonly used simple diluents such as water and liquid paraffin, including wetting agents, sweeteners, flavoring agents, and preservatives, among others. Formulations for non-oral administration include sterile solutions, non-aqueous solvents, suspensions, emulsions, freeze-dried preparations, and suppositories. Non-aqueous solvents and suspensions may include propylene glycol, polyethylene glycol, plant oils such as olive oil, and injectable esters such as ethyl oleate, among others. Bases for suppositories may include Witepsol, macrogol, Tween 61, cocoa butter, lauric acid, and glycerogelatin, among others.

The pharmaceutical composition according to the present disclosure is administered in an amount that is pharmaceutically effective. In the present disclosure, the term "pharmaceutically effective amount" refers to an amount sufficient to treat a disease, with a reasonable benefit/risk ratio applicable to medical treatment. The effective dosage level can be determined based on factors such as the type and severity of the patient's condition, the activity of the drug, sensitivity to the drug, timing of administration, route of administration, excretion rate, treatment duration, concomitant medications, and other factors well known in the medical field.

The pharmaceutical composition according to the present disclosure may be administered as a single therapeutic agent or in combination with other therapeutic agents, and may be administered sequentially or simultaneously with conventional therapies, either as a single dose or in multiple doses. Considering all the aforementioned factors, it is important to administer an amount that achieves maximum effect with minimal dosage and without side effects, which can be easily determined by a person skilled in the art to which the present disclosure pertains.

The pharmaceutical composition according to the present disclosure may be administered to an individual via various routes. All methods of administration are contemplated, including, for example, oral, intravenous, intramuscular, subcutaneous, and intraperitoneal injections.

The content of the compound in the composition of the present disclosure can be appropriately adjusted according to the symptoms of the disease, the degree of progression of the symptoms, the condition of the patient, and so on. For example, it may range from 0.0001 to 99.9 weight% based on the total weight of the composition, or from 0.001 to 50 weight%, but is not limited thereto. The aforementioned content ratio is based on the dry weight excluding the solvent.

The dosage of the pharmaceutical composition according to the present disclosure is selected based on considerations such as the individual's age, weight, sex, and physical condition. It is apparent that the concentration of the active ingredient included in the pharmaceutical composition can be varied depending on the subject, and preferably, it is included in the pharmaceutical composition at a concentration of 0.01 to 5,000 *µg*/ml. If the concentration is less than 0.01 *µg* /ml, it may not exhibit pharmacological activity, and if it exceeds 5,000 *µg* /ml, it may exhibit toxicity to the human body.

In the present disclosure, the term "individual" refers to a subject in need of treatment for a disease, and more specifically, it means mammals such as humans or non-human primates, mice, rats, dogs, cats, horses, and cattle.

In the present disclosure, the term "administration" means providing a specified composition of the present disclosure to an individual by any appropriate method.

In the present disclosure, "prevention" refers to any act that inhibits or delays the onset of the intended disease, and "treatment" refers to any act by which the symptoms of the intended disease and the associated metabolic abnormalities are improved or favorably altered by the administration of the pharmaceutical composition according to the present disclosure. Furthermore, "improvement" refers to any act that reduces parameters related to the intended disease, such as the severity of symptoms, through the administration of the composition according to the present disclosure.

According to an exemplary embodiment of the present disclosure, the isocquinoline derivative compound may be represented by the following Chemical Formula 1 to Chemical Formula 6.

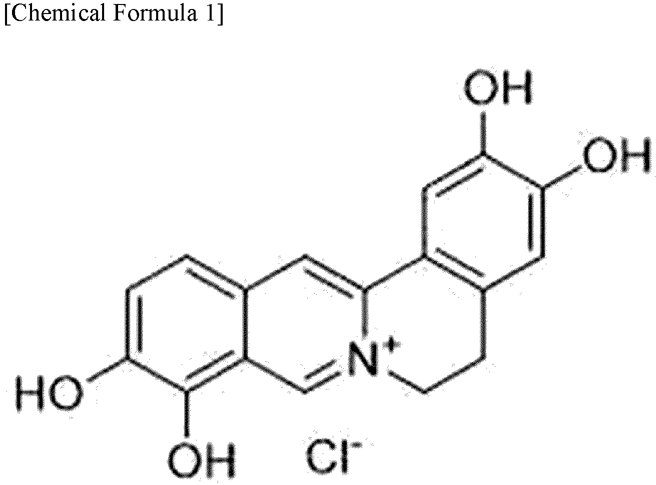

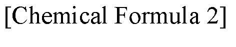

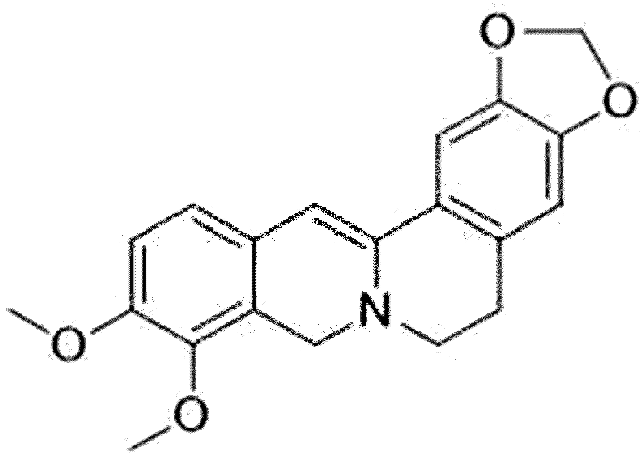

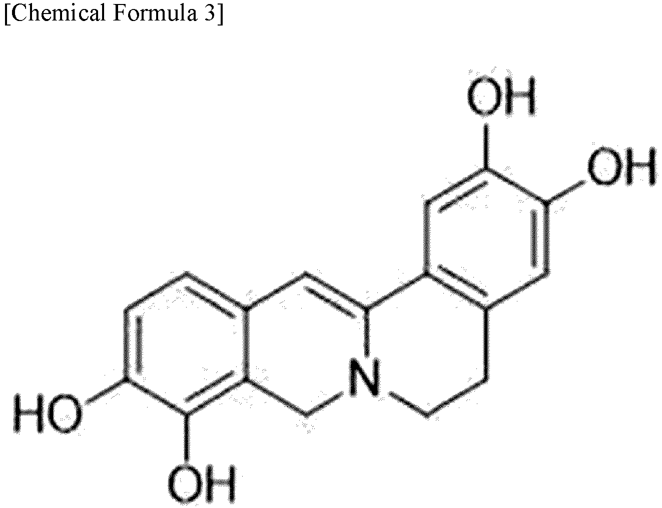

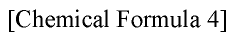

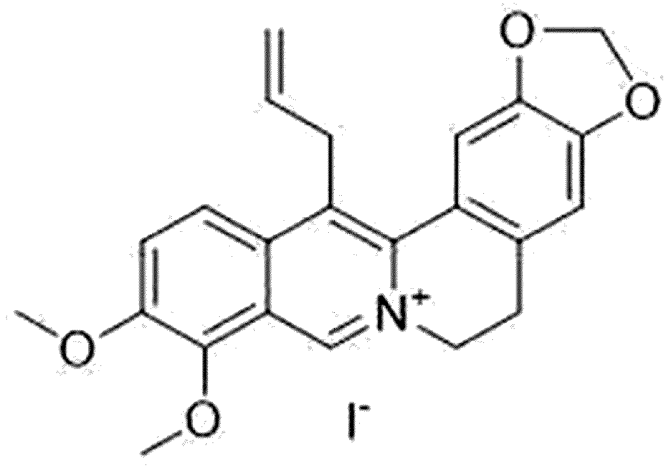

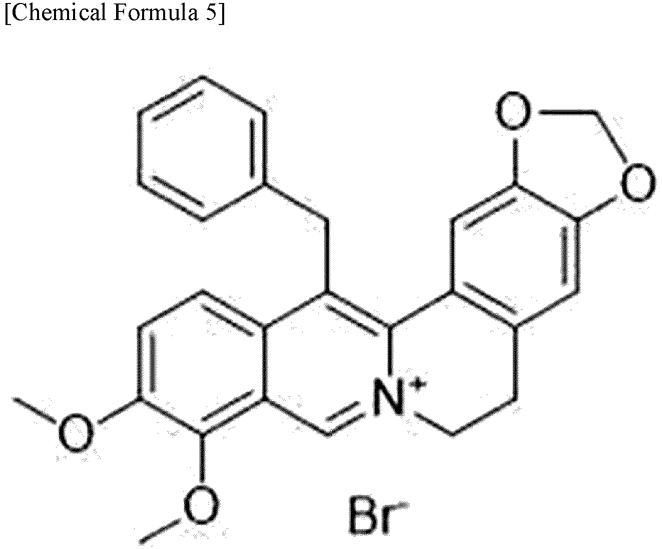

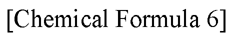

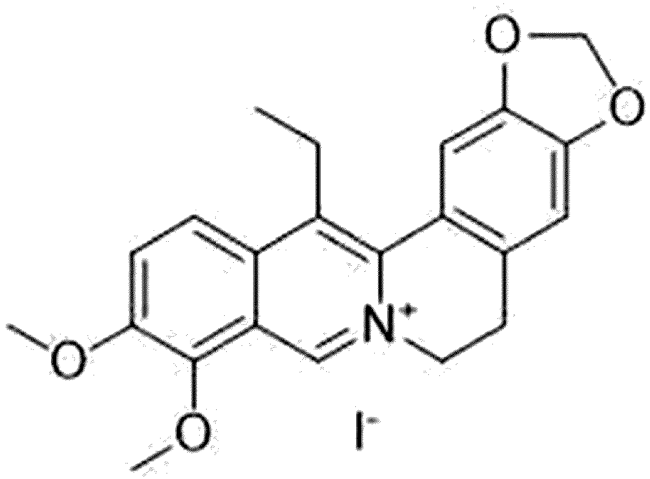

In one exemplary embodiment, the pharmaceutically acceptable salt is preferably an acid addition salt formed from a pharmaceutically acceptable organic acid. The term "pharmaceutically acceptable" used in the present disclosure means a characteristic that indicates non-toxicity to cells or humans exposed to the compound or its acid addition salt. The acid addition salt is obtained from inorganic acids such as hydrochloric acid, nitric acid, phosphoric acid, sulfuric acid, hydrobromic acid, hydroiodic acid, nitrous acid, or arsenic acid, and from non-toxic organic acids such as aliphatic mono- and dicarboxylates, phenyl-substituted alkanoylates, hydroxyalkanoates, alkanedioates, aromatic acids, and aliphatic and aromatic sulfonic acids. Examples of such pharmacologically non-toxic salts include sulfates, pyrosulfates, bisulfates, sulfites, bisulfites, nitrates, phosphates, mono hydrogen phosphates, dihydrogen phosphates, metaphosphates, pyrophosphates, chlorides, bromides, iodides, fluorides, acetates, propionates, decanoates, caprylates, acrylates, formates, isobutyrates, caprates, heptanoates, propionolates, oxalates, malonates, succinates, suberates, sebacates, fumarates, maleates, butan-1,4-dioates, hexan-1,6-dioates, benzoates, chlorobenzoates, methylbenzoates, dinitrobenzoates, hydroxybenzoates, methoxybenzoates, phthalates, terephthalates, benzenesulfonates, toluenesulfonates, chlorobenzenesulfonates, xylensulfonates, phenylacetates, phenylpropionates, phenylbutyrates, citrates, lactates, hydroxybutyrates, glycolates, maleates, tartrates, methanesulfonates, propansulfonates, naphthalene-1-sulfonates, naphthalene-2-sulfonates, or mandelates.

The acid addition salts according to the present disclosure can be prepared by conventional methods, for example, by dissolving the compounds represented by Chemical Formula 1 to Chemical Formula 6 in a large excess of acidic aqueous solution and precipitating the salts using a water-miscible organic solvent such as methanol, ethanol, acetone, or acetonitrile. Additionally, the mixture can be dried by evaporating the solvent or excess acid, or the precipitated salt can be prepared by suction filtration.

Additionally, a pharmaceutically acceptable metal salt can be prepared using a base. Alkali metal or alkaline earth metal salts can be obtained by dissolving the compound in an excess of alkali metal hydroxide or alkaline earth metal hydroxide solution, filtering the insoluble compound salt, and evaporating and drying the supernatant. At this time, it is pharmaceutically appropriate to produce metal salts such as sodium, potassium, or calcium salts. Additionally, the corresponding silver salts can be obtained by reacting alkaline metal or alkaline earth metal salts with a suitable anion (e.g., silver nitrate).

In one exemplary embodiment, the compounds represented by the above chemical formulas 1 to 6 of the present disclosure may include not only their stereoisomers and pharmaceutically acceptable salts but also any solvates, hydrates, racemates, or prodrugs that can be derived therefrom. In particular, the compounds represented by the above chemical formulas 1 to 6 may include isomers at all asymmetric carbons. Moreover, when the compounds of the present disclosure have an asymmetric carbon center in their substituents, they may exist as R or S isomers, racemates, diastereomers, isomer mixtures, and individual diastereomeric isomers, and all of these isomers and their mixtures are included within the scope of the present disclosure.

According to an exemplary embodiment of the present disclosure, the isoquinoline derivative compounds may enhance mitophagy activity.

According to an exemplary embodiment of the present disclosure, the enhancement of mitophagy activity may involve the removal of dysfunctional mitochondria.

According to an exemplary embodiment of the present disclosure, the dysfunctional mitochondria may induce a neurodegenerative disease, and the neurodegenerative disease may be selected from the group consisting of Alzheimer's disease, Parkinson's disease, amyotrophic lateral sclerosis, Huntington's disease, multiple sclerosis, immune-mediated brain dysfunction, progressive neurodegenerative disease, metabolic brain disease, Niemann-Pick disease, cerebral ischemia, and dementia due to cerebral hemorrhage, and preferably may be Alzheimer's disease or dementia, but is not limited thereto.

Additionally, another object of the present disclosure is to provide a food composition for promoting mitophagy, comprising isoqinoline derivative compounds or salts thereof as an active ingredient. The salt may be a food-grade acceptable salt. Additionally, the food composition includes a health functional food composition.

The term "improvement" as used in the present disclosure means any action that at least reduces parameters related to the condition being treated, such as the severity of symptoms.

The food composition of the present disclosure may contain, in addition to the active ingredient of the present disclosure, various flavoring agents or natural carbohydrates as additional components, similar to conventional food compositions.

Examples of the aforementioned natural carbohydrates include monosaccharides, such as glucose and fructose; disaccharides, such as maltose and sucrose; and polysaccharides, such as dextrin and cyclodextrin, as well as conventional sugars and sugar alcohols such as xylitol, sorbitol, and erythritol. The aforementioned flavoring agents may advantageously include natural flavoring agents (taumatine), stevia extracts (for example, rebaudioside A, glycyrrhizin, etc.), and synthetic flavoring agents (such as saccharin and aspartame). The food composition of the present disclosure may be formulated in the same manner as the pharmaceutical composition for use as a functional food or can be added to various foods. Examples of foods to which the composition of the present disclosure can be added include, but are not limited to, beverages, meats, chocolates, food products, snacks, pizza, noodles, other pasta products, gums, candies, ice creams, alcoholic beverages, vitamin complexes, and health supplements.

Additionally, the food composition may contain, besides the active ingredient extract, various nutrients, vitamins, minerals (electrolytes), synthetic flavoring agents and natural flavoring agents, coloring agents, and emulsifiers (such as cheese and chocolate), pectin and a salt thereof, alginic acid and a salt thereof, organic acids, protective colloid thickeners, pH regulators, stabilizers, preservatives, glycerin, alcohol, and carbonating agents used in carbonated beverages. Furthermore, the food composition of the present disclosure may contain pulp for the production of natural fruit juices, fruit juice beverages, and vegetable beverages.

The functional food composition of the present disclosure may be manufactured and processed in forms such as tablets, capsules, powders, granules, liquids, and lozenges for the purpose of promoting mitophagy activity. In the present disclosure, the term "health functional food composition" refers to food manufactured and processed using raw materials or components with beneficial functionality for the human body, as defined by the Health Functional Food Act No. 6727, and is intended for consumption to achieve beneficial effects for health purposes, such as regulating nutrients related to the structure and function of the human body or for physiological effects. The health functional food of the present disclosure may contain conventional food additives, and the suitability of these additives is determined based on the specifications and standards related to the respective items according to the general provisions and general testing methods outlined in the Food and Drug Administration's approved food additive regulations, unless otherwise specified. Items listed in the "Food Additive Regulations" include, for example, chemically synthesized substances such as ketones, glycine, calcium citrate, nicotinic acid, and cinnamic acid; natural additives such as colorants, licorice extract, crystalline cellulose, caramel color, and guar gum; and mixed formulations such as sodium L-glutamate preparations, alkaline agents for noodle additives, and preservative preparations, as well as tartrazine preparations. For example, health functional foods in tablet form can be granulated by mixing the active ingredients of the present disclosure with excipients, binders, disintegrants, and other additives using conventional methods, and then compressed using lubricants, or the mixture can be directly compressed into tablets. Additionally, the tablet form of the health functional food may contain glidants or other similar agents as needed. Hard capsule forms of health functional foods can be produced by filling conventional hard capsules with a mixture of the active ingredients of the present disclosure and additives such as excipients. Soft capsule forms can be produced by filling gelatin or similar capsule materials with a mixture of the active ingredients and additives such as excipients. The soft capsule forms may contain plasticizers such as glycerin or sorbitol, as well as coloring agents and preservatives, as needed. Lozenge forms of health functional foods can be prepared by molding a mixture of the active ingredients of the present disclosure with excipients, binders, disintegrants, etc., using methods known in the art. Additionally, they may be coated with powdered sugar or other coating agents as needed, or coated on the surface with substances such as starch or talc. Granulated forms of health functional foods can be produced by forming granules from a mixture of the active ingredients of the present disclosure with excipients, binders, disintegrants, etc., using methods known in the art. Additionally, they may contain flavoring agents, glidants, or other additives as needed.

When using the compounds, their derivatives, or food-grade acceptable salts of the present disclosure as food additives, the compounds can be added directly or used in conjunction with other foods or food ingredients, and they can be appropriately utilized according to conventional methods. The mixing amount of the active ingredient can be appropriately determined according to the intended use (prevention, health, or therapeutic treatment). Generally, when manufacturing food or beverages, the compounds of the present disclosure may be added in an amount of 15 weight% or less, or 10 weight% or less, based on the total weight of the raw materials. However, for long-term intake aimed at health and hygiene or health regulation, the amount may be below the specified range, and since there are no safety concerns, the active ingredient may also be used in amounts exceeding this range.

There are no specific restrictions on the types of food. Examples of foods that may contain the aforementioned substances include meats, sausages, bread, chocolates, candies, snacks, cookies, pizza, noodles, other pasta products, gums, dairy products such as ice creams, various soups, beverages, teas, drink mixes, alcoholic beverages, and vitamin complexes, encompassing all forms of health functional foods in the conventional sense.

The health beverage composition according to the present disclosure may contain various flavoring agents or natural carbohydrates as additional components, similar to conventional beverages. The aforementioned natural carbohydrates include monosaccharides such as glucose and fructose; disaccharides such as maltose and sucrose; polysaccharides such as dextrin and cyclodextrin; and sugar alcohols such as xylitol, sorbitol, and erythritol. Sweeteners may include natural sweeteners such as taumatine and stevia extracts, as well as synthetic sweeteners such as saccharin and aspartame. The proportion of the natural carbohydrates is typically about 0.01-0.20 g, or about 0.04-0.10 g per 100 mL of the composition of the present disclosure.

In addition, the composition of the present disclosure may contain various nutrients, vitamins, electrolytes, flavoring agents, coloring agents, pectin and a salt thereof, alginic acid and a salt thereof, organic acids, protective colloid thickeners, pH regulators, stabilizers, preservatives, glycerin, alcohol, and carbonating agents used in carbonated beverages. Additionally, the composition of the present disclosure may contain pulp for the production of natural fruit juices, fruit juice beverages, and vegetable beverages. These ingredients may be used independently or in combination. The ratio of these additives is not critically important, but it is generally selected within the range of 0.01 to 0.20 weight parts per 100 weight parts of the composition of the present disclosure.

In this specification, "health functional food" refers to the same term as food for special health use (FoSHU), meaning food processed to efficiently exhibit biological regulation functions in addition to providing nutrition, which has significant medical and health benefits. Such food can be manufactured in various forms, including tablets, capsules, powders, granules, liquids, and lozenges, to achieve beneficial effects for the prevention or improvement of peripheral neuropathy.

The health functional food of the present disclosure can be manufactured using methods commonly employed in the industry, and during production, raw materials and ingredients typically added in the industry can be included. Additionally, unlike conventional pharmaceuticals, health functional foods are made from food ingredients, which offers the advantage of having no side effects that may occur with long-term use of medications, and they may also provide excellent portability.

Additionally, the present disclosure provides a pharmaceutical composition for the prevention or treatment of diseases caused by mitochondrial dysfunction, comprising isoqinoline derivatives or salts thereof as an active ingredient.

According to an exemplary embodiment of the present disclosure, the disease caused by mitochondrial dysfunction may be a neurodegenerative disease.

Additionally, the present disclosure provides a food composition for the prevention or improvement of diseases caused by mitochondrial dysfunction, comprising isoqinoline derivatives or salts thereof as an active ingredient.

Additionally, the present disclosure provides a method for increasing the activity of mitophagy in cells, comprising the step of treating cells with isoqinoline derivative compounds in vitro.

The cells of the present disclosure may be cells expressing mt-Keima, and "mt-Keima" refers to a coral-derived fluorescent protein that exhibits pH-dependent changes in fluorescent characteristics (Katayama H et al, 2011, Chem Biol). mt-Keima exhibits significantly enhanced fluorescence signals at 458 nm in neutral conditions (pH 7) and shows strong fluorescence at 561 nm under acidic conditions (pH 4) due to changes in protein structure. By utilizing these characteristics, mt-Keima can be expressed specifically in mitochondria, and by performing integrated analysis of the fluorescence signals at 458 nm and 561 nm, the fluorescence signal can be analyzed when mitochondria in a neutral environment (pH = 7.4-7.8) are converted to an acidic environment (pH = 4.5) by lysosomes delivered from mitophagy, allowing for quantitative measurement of mitophagy activity.

Additionally, the present disclosure provides a method for inhibiting mitochondrial dysfunction, comprising the step of treating cells with isoqinoline derivative compounds in vitro.

Unless otherwise specified, all numbers, values, and/or expressions representing components, reaction conditions, and ingredient contents used in this specification are to be understood as approximations reflecting various uncertainties in measurements that arise in obtaining such values among fundamentally different ones, and should be qualified in all cases by the term "about." Additionally, when a numerical range is disclosed in this specification, such range is continuous, and unless otherwise noted, it includes all values from the minimum to the maximum value stated, inclusive of the maximum. Furthermore, when such a range refers to integers, unless otherwise noted, it includes all integers from the minimum value to the maximum value stated, inclusive of the maximum.

For example, in this specification, when a range is described for a variable, the variable is to be understood as encompassing all values within the described range, including the stated endpoints of that range. For example, the range of "5 to 10" includes not only the values 5, 6, 7, 8, 9, and 10 but also any subrange such as 6 to 10, 7 to 10, 6 to 9, 7 to 9, and so forth. It is also understood to include any valid integer values between the stated range, such as 5.5, 6.5, 7.5, as well as subranges like 5.5 to 8.5 and 6.5 to 9. Additionally, for example, the range of "10% to 30%" includes not only the integer values of 10%, 11%, 12%, 13%, up to 30%, but also any subrange such as 10% to 15%, 12% to 18%, and 20% to 30%. It is also understood to include any valid values within the stated range, such as 10.5%, 15.5%, and 25.5%.

Hereinafter, the present disclosure will be described in more detail by way of examples. These examples are provided solely to illustrate the present disclosure in more detail, and it will be apparent to those skilled in the art that the scope of the invention is not limited to these examples.

### <Example 1> Synthesis of Isoquinoline Derivatives

Isoquinoline derivatives of the present disclosure, CD1-271, CD1-363, CD1-367, CD1-386, CD1-404, and CD1-409, were synthesized.

### <1-1> Synthesis of CD1-271

The precursor, CD1-012 (1g, 2.6mmol), was dissolved in methanol (30ml) in a dried round-bottom flask, and0.5M hydrochloric acid aqueous solution (8ml) was added at 0°C and stirred for 6 hours. Afterwards, it was concentrated under reduced pressure to obtain CD1-271 with a 99% yield.

### <1-2> Synthesis of CD1-363

A precursor, berberine (0.5g, 1.2mmol), was dissolved in methanol (20ml) in a dried round-bottom flask, after which potassium carbonate (0.5g, 3.6mmol) was added. Thereafter, a 2.2M sodium borohydride aqueous solution (0.6ml) containing 5% sodium hydroxide was added at 0 °C and stirred for 3 hours. The resulting solid was filtered, washed with distilled water, and dried under vacuum, yielding compound CD1-363 with an 84% yield.

### <1-3> Synthesis of CD1-367

A synthesis was carried out using the same method as in Example 1-1, with CD363 as a precursor, yielding CD1-367 with a 96% yield.

### <1-4> Synthesis of CD1-386, CD1-404, and CD1-409

The precursor, berberine (0.5g, 1.3mmol), was dissolved in a 5 N sodium hydroxide aqueous solution (20ml) in a dried round-bottom flask, after which acetone (5ml) was added at 0°C, and stirred for 6 hours. After concentration under reduced pressure, cold water (30ml) was added, and the resulting solid was filtered. The filtered solid was washed with distilled water, and then dried under vacuum. The dried solid (0.4g, 1 mmol) was dissolved in anhydrous CH₂CL₂ (10ml) in a pressure tube, after which allyl iodide (0.4g, 1 mmol) was added, and the mixture was stirred at 100°C 4 hours. After this, mixture was concentrated under reduced pressure, and the obtained mixture was adsorbed onto silica gel and separated by silica gel chromatography purification (CH₂Cl₂: acetone = 50:1 → 15:1 v/v) to obtain compound CD1-386 with a yield of 35%. In addition, CD1-404 was obtained with a yield of 14%, and CD1-409 was obtained with a yield of 47%.

### <1-5> NMR Analysis

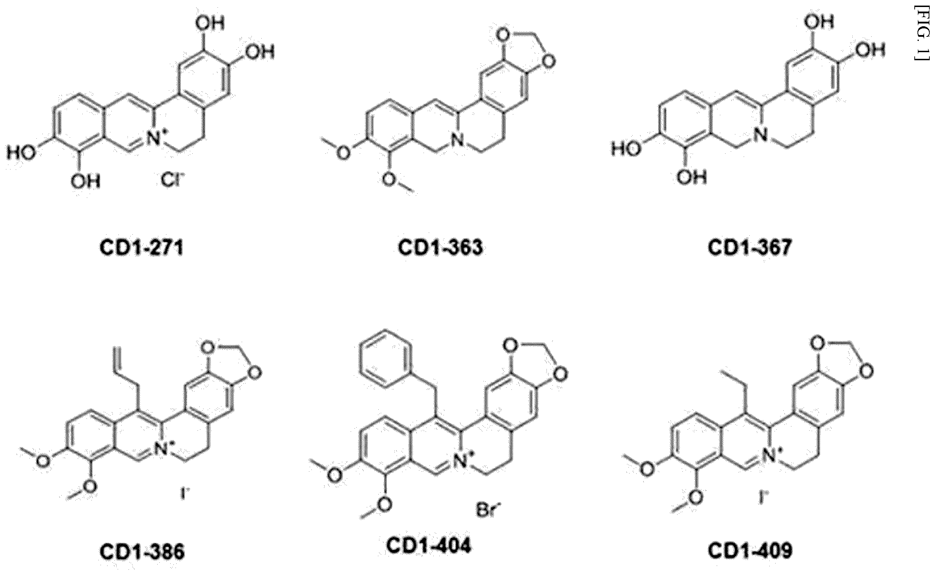

NMR analysis was performed to confirm the structures of the compounds obtained in Examples 1-1 to 1-4. The results are shown below, and the analyzed structures are shownin Figure 1.
CD1-271: ¹H NMR (DMSO-*d*₆, 400 MHz) δ 9.72 (s, 1H), 8.55 (s, 1H), 7.75 (d, *J* = 8.8 Hz, 1H), 7.47 (s, 1H), 4.83 (t, J = 6.0 Hz, 2H), 3.09 (t, J = 6.4 Hz, 2H)
CD1-363: ¹H NMR (DMSO-*d*₆, 400 MHz) δ 7.29 (s, 1H), 6.83 (d, *J* = 8.4 Hz, 1H), 6.75 (s, 1H), 6.70 (d, *J* = 8.4 Hz, 1H), 6.04 (s, H), 6.00 (s, 2H), 4.20 (s, 2H), 3.75 (s, 3H), 3.71(s, 3H), 3.36 (t, *J* = 6.0 Hz, 2H), 2.80 (t, *J* = 6.0 Hz, 2H)
CD1-367: ¹H NMR (MeOH-*d*₄, 400 MHz) δ 7.48 (s, 1H), 6.83-6.91 (m, 3H), 6.74 (d, J = 8.4 Hz, 1H), 4.12 (t, J = 8.0 Hz, 2H), 3.12 (t, J = 8.0 Hz, 2H)
CD1-386: ¹H NMR (CDCl₃, 400 MHz) δ 10.39 (s, 1H), 7.81 (s, 2H), 7.36 (s, 1H), 6.87 (s, 1H), 6.41-6.34 (m, 1H), 6.08 (s, 2H), 5.45 (d, J = 10.8 Hz, 1H), 5.15 (br, 2H), 4.96 (d, J = 17.6 Hz, 1H), 4.40 (s, 3H), 4.06 (s, 3H), 4.00 (br, 2H), 3.27 (t, J = 5.6 Hz, 2H)
CD1-404: ¹H NMR (MeOH-*d*₄, 400 MHz) δ 10.02 (s, 1H), 8.09 (d, J = 9.2 Hz, 1H), 7.79 (d, J = 9.6 Hz, 1H), 7.37 (t, J = 7.6 Hz, 2H), 7.28 (d, J = 6.4 Hz, 1H), 7.15 -7.14 (m, 3H), 6.94 (s, 1H), 6.06 (s, 2H), 4.85 (br, 2H), 4.73 (s, 2H), 4.01 (s, 3H), 4.00 (s, 3H), 3.15 (t, J = 5.6 Hz, 2H)
CD1-409: ¹H NMR (DMSO-*d*₆, 400 MHz) δ 9.90 (s, 1H), 8.25-8.18 (m, 2H), 7.30 (s, 1H), 7.16 (s, 1H), 6.19 (s, 2H), 4.80 (br, 2H), 4.09 (s, 6H), 3.38 (m, 2H), 3.10 (t, J = 5.2 Hz, 2H), 1.48-1.43 (m, 3H)

### <Example 2> Analysis of Mitophagy Activity Promotion of Isoquinoline Derivatives

The isoquinoline derivatives of the present disclosure were analyzed to determine whether they promote the activity of mitophagy. Specifically, a mitokeyma fluorescent protein quantification method, which allows for the quantitative measurement of mitophagy activity in living cells, was used. The isoquinoline derivatives prepared in Example 1 were each treated at a concentration of 20 µM for 24 hours in the human normal lung cell line BEAS-2B. After treatment, the activity of mitophagy was measured using a flow cytometer. As a negative control, a control group (Control, con) without compound treatment was used, and as a positive control, CCCP (10 µM), which experimentally induces mitophagy activity, and CD1-012, a precursor of the compound of the present disclosure known to induce mitophagy activity, were used.

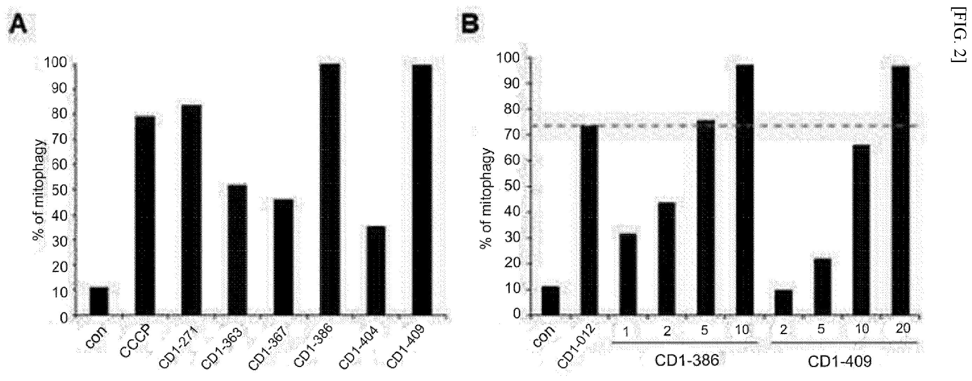

As a result, as shown in Figure 2, the isoquinoline derivatives of the present disclosure were found to significantly increase mitophagy activity compared to the con group (Figure 2A). Additionally, when the mitophagy activity promotion ability of CD1-386 and CD1-409, which exhibited the best mitophagy activity promotion, was compared with that of CD1-012, CD1-012 showed maximum mitophagy activity at a concentration of 20 µM, whereas CD1-386 showed maximum mitophagy activity at 5 µM and CD1-409 at 10 µM. Therefore, it was confirmed that CD1-386 has 4 times and CD1-409 has 2 times higher activity than CD1-012, the precursor of the isoquinoline derivatives.

### <Example 3> Confirmation of Mitochondrial Toxicity of Isoquinoline Derivatives

CCCP, which experimentally promotes mitophagy activity, induces mitophagy activity but also causes mitochondrial dysfunction and toxicity. Therefore, the novel isoquinoline derivatives of the present disclosure were examined to determine whether they induce mitochondrial dysfunction and toxicity. Specifically, mitochondrial membrane potential, known as a mitochondrial function indicator, was analyzed using the TMRM (tetramethylrhodamine methyl ester) assay.

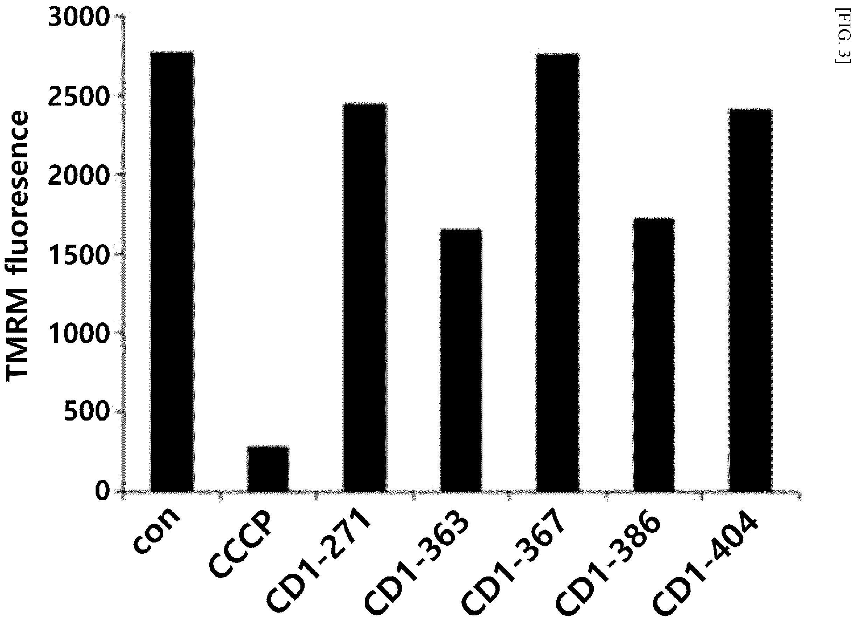

As a result, as shown in Figure 3, the mitochondrial membrane potential in mitochondria treated with 10 µM CCCP was completely reduced, while the mitochondrial membrane potential in mitochondria treated with 20 µM of the isoquinoline derivatives of the present disclosure did not decrease significantly. Therefore, it was confirmed that the novel isoquinoline derivatives of the present disclosure do not induce mitochondrial dysfunction or toxicity.

### <Example 4> Confirmation of Cytotoxicity of Isoquinoline Derivatives

A colony forming assay was performed to confirm the cytotoxicity of the novel isoquinoline derivatives of the present disclosure. Specifically, the isoquinoline derivatives were treated to the human normal lung cell line BEAS-2B for 24 hours, followed by culturing for 2 weeks to analyze the colony formed. As controls, a control group (con) that was not treated with anything and a positive control treated with CCCP, which induces mitophagy activity but also causes mitochondrial dysfunction and toxicity, were used.

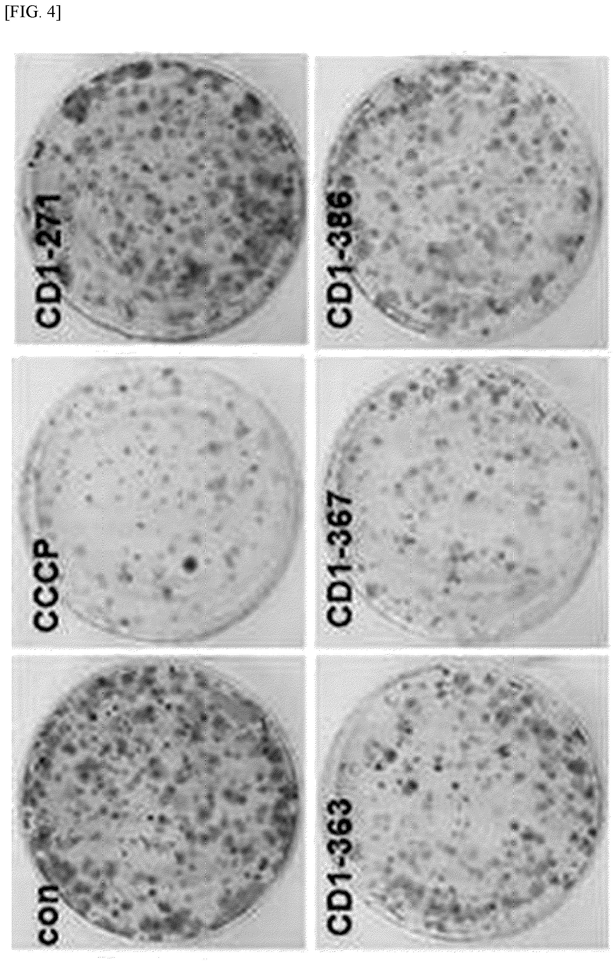

As a result, as shown in Figure 4, the cells treated with CCCP exhibited a decrease in both the size and number of colonies. However, the isoquinoline derivatives of the present disclosure, except for CD1-367, showed colony sizes and numbers similar to the con group, confirming that the derivatives, except for CD1-367, did not exhibit cytotoxicity.

Therefore, the present disclosure has synthesized novel isoquinoline derivatives, CD1-271, CD1-363, CD1-367, CD1-386, CD1-404, and CD1-409, and it has been confirmed that these novel isoquinoline derivatives promote the activity of mitophagy. Additionally, it was confirmed that these derivatives do not induce mitochondrial dysfunction and are safe derivatives that do not exhibit toxicity to cells.

### [Industrial Applicability]

The present disclosure relates to novel isoquinoline derivatives. The derivatives have an effect of promoting mitophagy activity, thereby reducing mitochondria with structural and/or functional damage. In particular, the novel isoquinoline derivatives according to the present disclosure do not induce mitochondrial dysfunction and have been identified as safe compounds that are non-toxic to cells, and thus can be utilized for the prevention, amelioration, and/or treatment of various diseases that may be caused by abnormal mitochondria.

## Claims

1. A pharmaceutical composition for promoting mitophagy activity, comprising an isoquinoline derivative compound or a salt thereof as an active ingredient.

2. The pharmaceutical composition of claim 1, wherein the isoquinoline derivative compound is represented by the following Chemical Formula 1 to Chemical Formula 6.

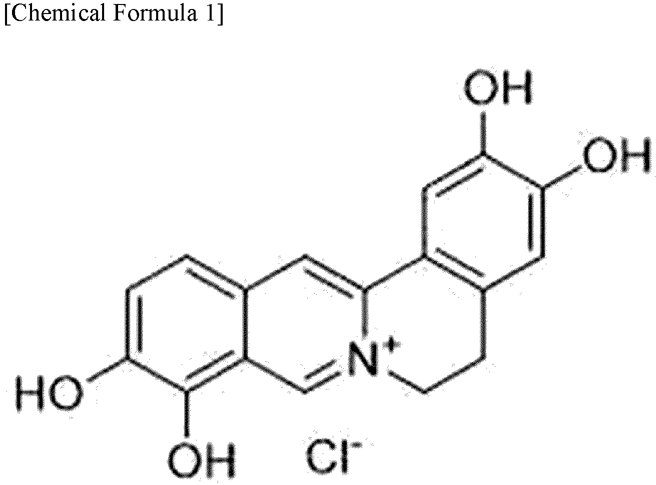

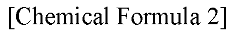

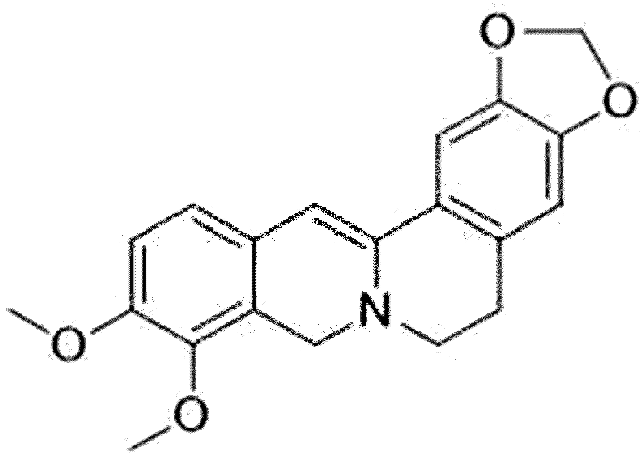

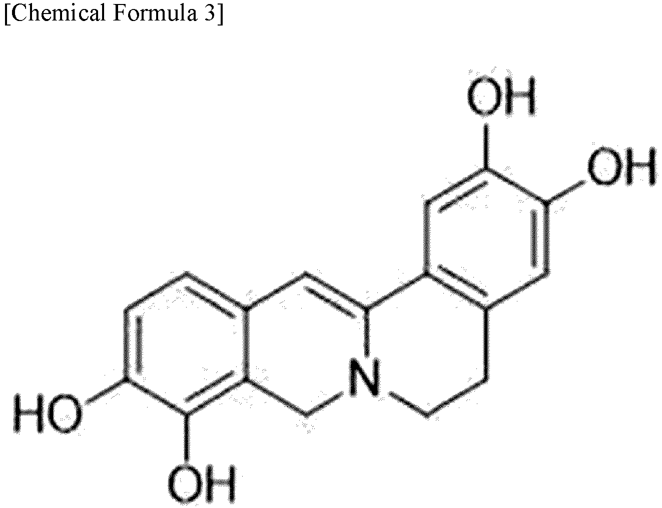

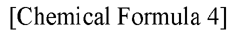

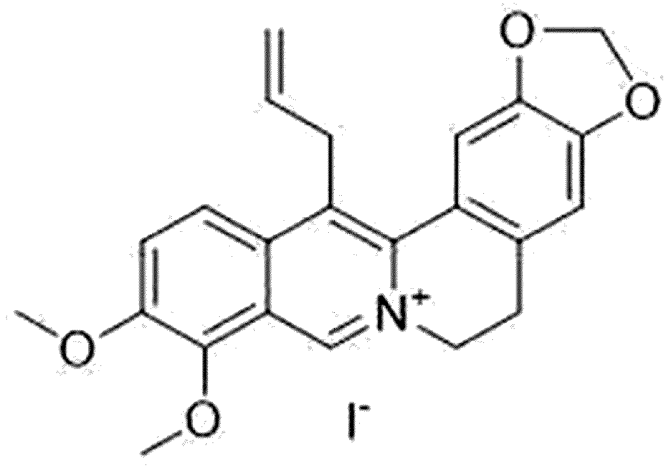

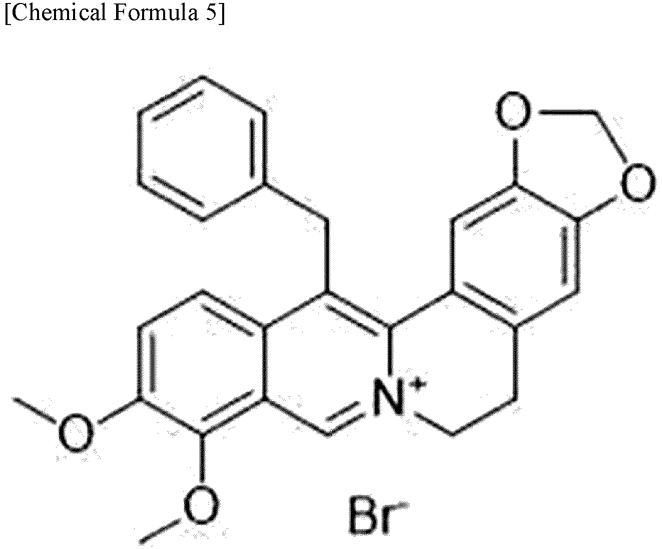

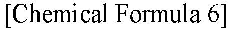

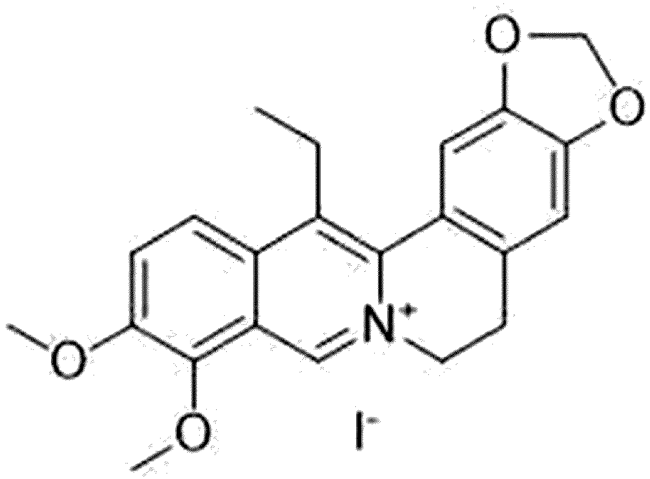

3. The pharmaceutical composition of claim 1, wherein the isoquinoline derivative compound increases mitophagy activity.

4. The pharmaceutical composition of claim 3, wherein the increase in mitophagy activity removes dysfunctional mitochondria.

5. The pharmaceutical composition of claim 4, wherein the dysfunctional mitochondria induce neurodegenerative disease.

6. The pharmaceutical composition of claim 5, wherein the neurodegenerative disease is selected from the group consisting of Alzheimer's disease, Parkinson's disease, amyotrophic lateral sclerosis, Huntington's disease, multiple sclerosis, immune-mediated brain dysfunction, progressive neurodegenerative disease, metabolic brain disease, Niemann-Pick disease, cerebral ischemia, and dementia due to cerebral hemorrhage.

7. The pharmaceutical composition of claim 6, wherein the neurodegenerative disease is Alzheimer's disease or dementia.

8. A food composition for promoting mitophagy activity, comprising an isoquinoline derivative compound or a salt thereof as an active ingredient.

9. The food composition of claim 8, wherein the isoquinoline derivative compound is represented by the following Chemical Formula 1 to Chemical Formula 6.

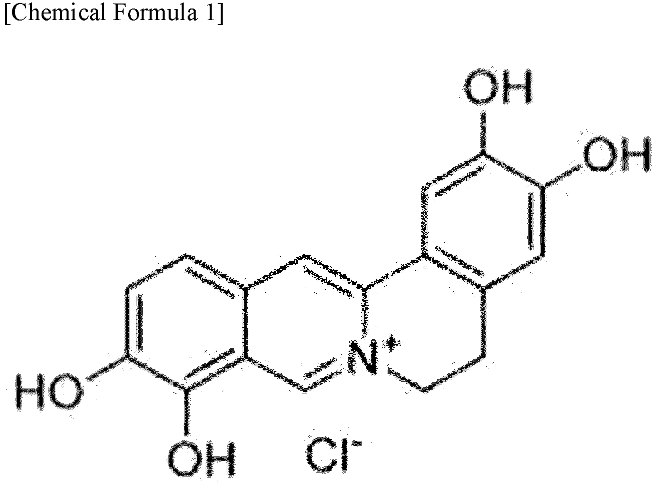

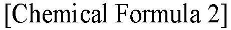

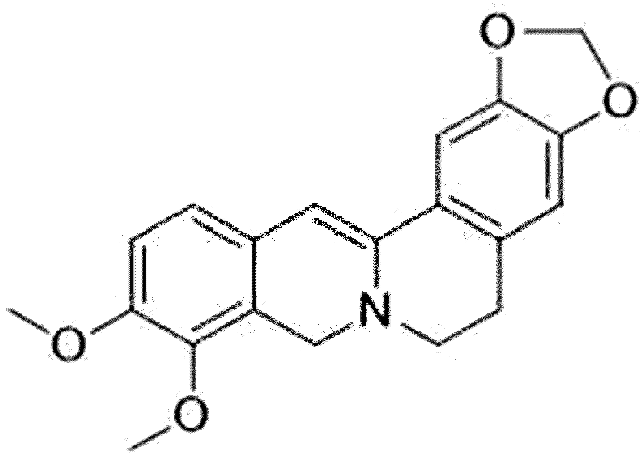

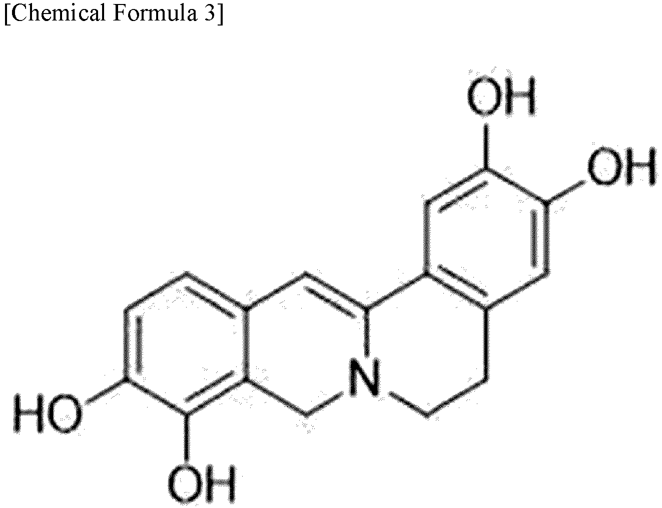

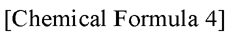

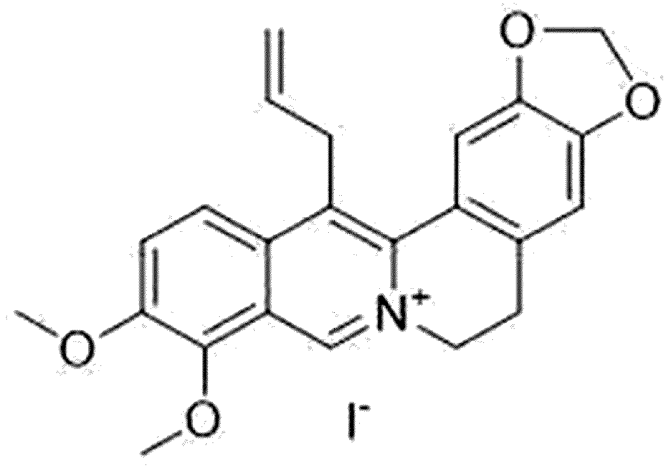

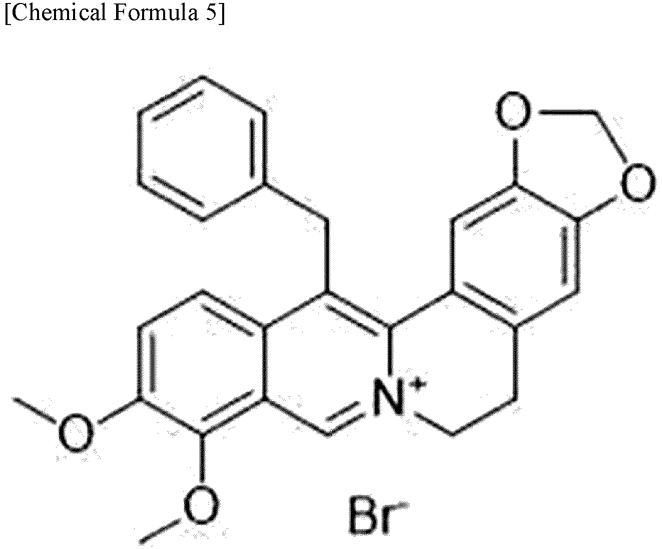

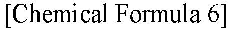

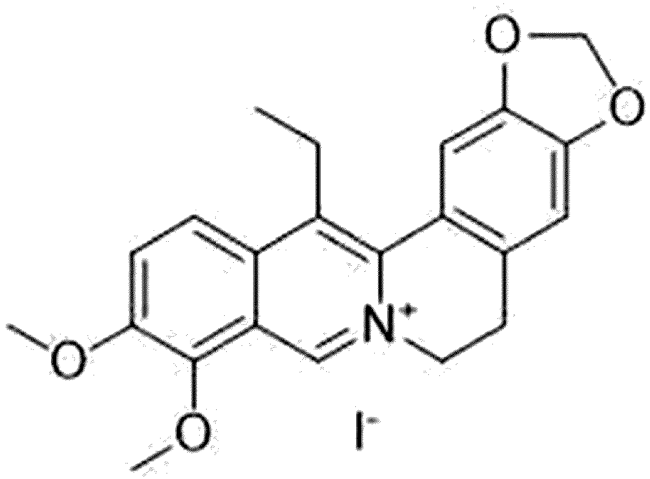

10. A pharmaceutical composition for the prevention or treatment of disease caused by mitochondrial dysfunction, comprising an isoquinoline derivative compound or a salt thereof as an active ingredient.

11. The pharmaceutical composition of claim 10, wherein the isoquinoline derivative compound is represented by the following Chemical Formula 1 to Chemical Formula 6.

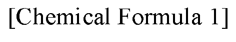

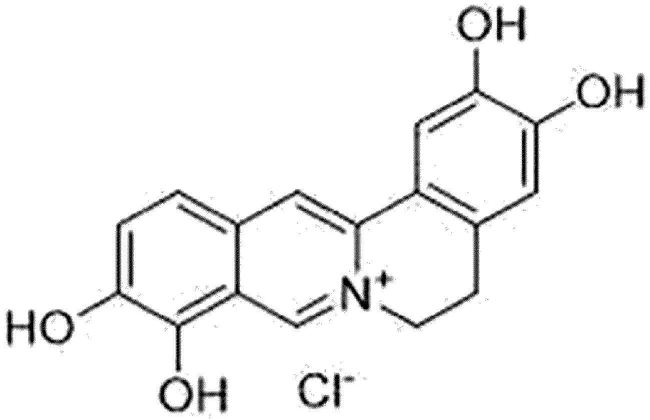

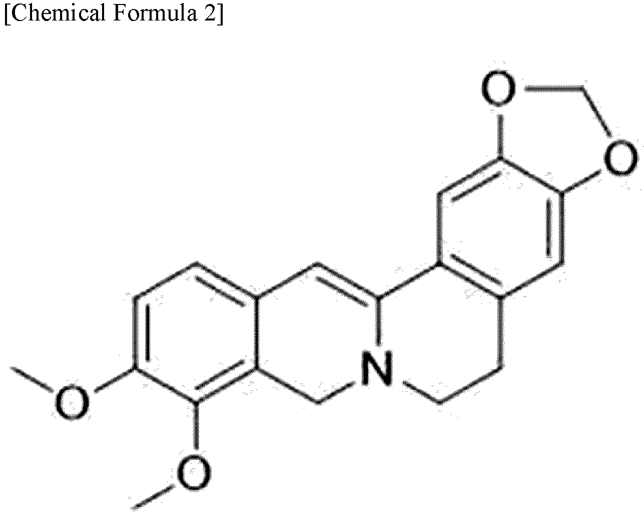

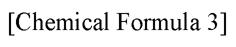

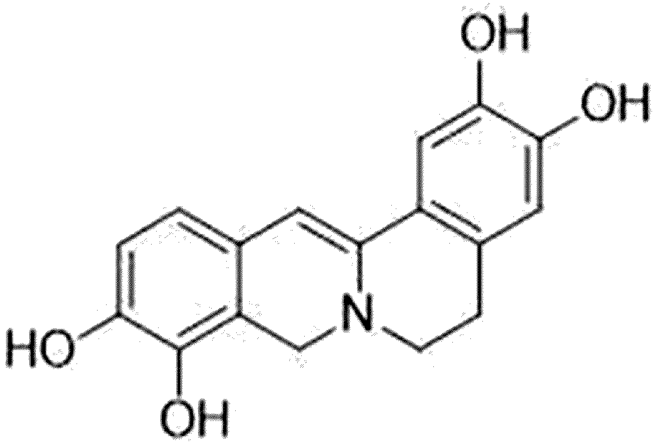

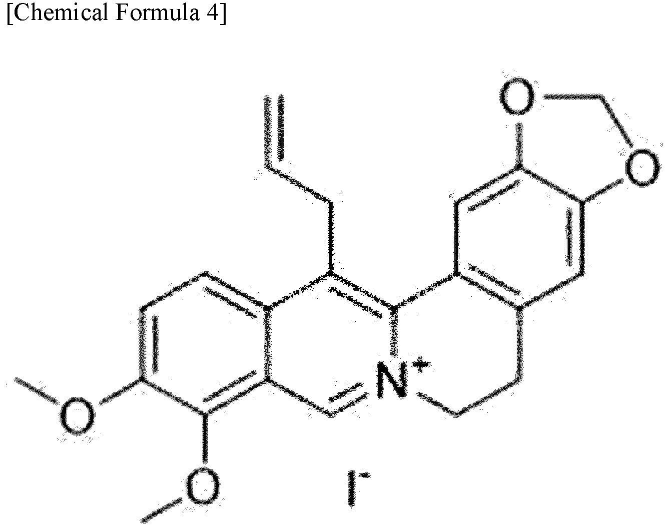

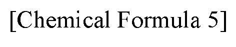

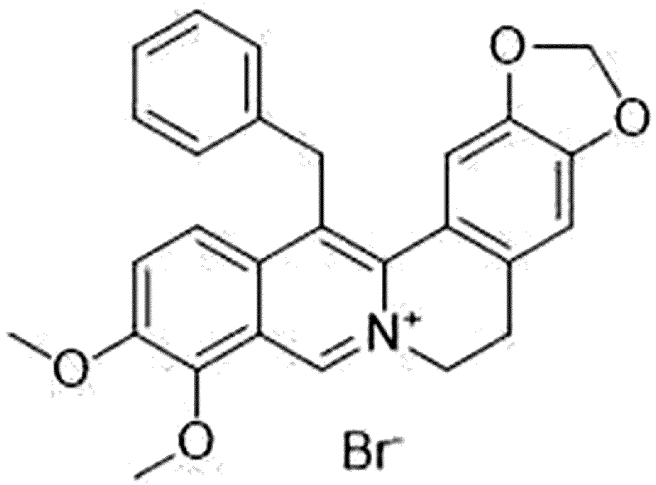

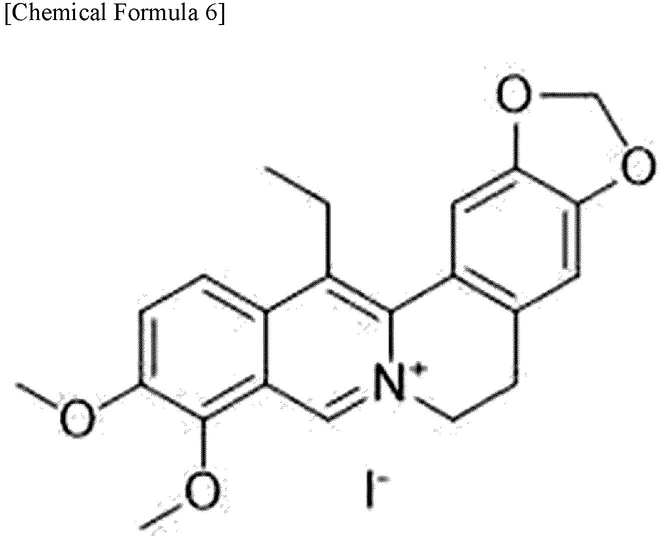

12. The pharmaceutical composition of claim 10, wherein the isoquinoline derivative compound increases mitophagy activity.

13. The pharmaceutical composition of claim 10, wherein the disease caused by mitochondrial dysfunction is a neurodegenerative disease.

14. The pharmaceutical composition of claim 13, wherein the neurodegenerative disease is selected from the group consisting of Alzheimer's disease, Parkinson's disease, amyotrophic lateral sclerosis, Huntington's disease, multiple sclerosis, immune-mediated brain dysfunction, progressive neurodegenerative disease, metabolic brain disease, Niemann-Pick disease, cerebral ischemia, and dementia due to cerebral hemorrhage.

15. A food composition for the prevention or improvement of disease caused by mitochondrial dysfunction, comprising an isoquinoline derivative compound or a salt thereof as an active ingredient.

16. The food composition of claim 15, wherein the isoquinoline derivative compound is represented by the following Chemical Formula 1 to Chemical Formula 6.

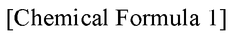

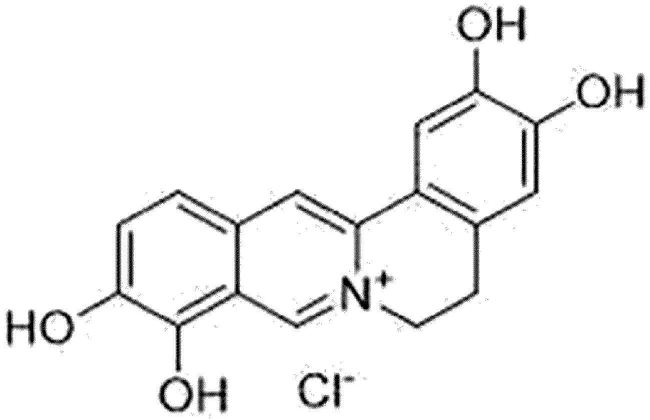

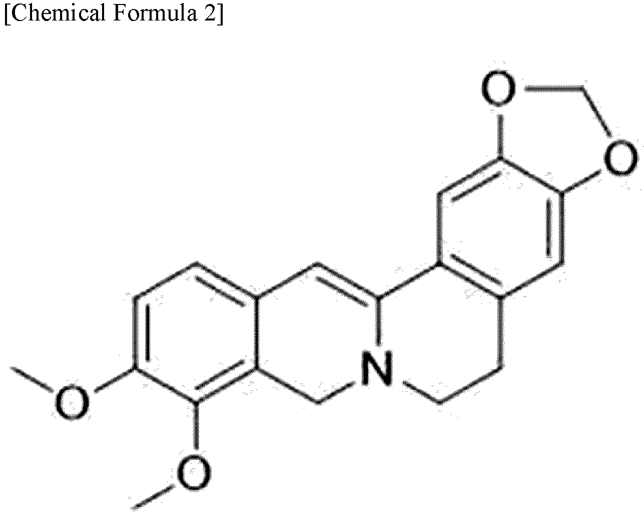

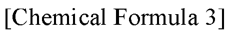

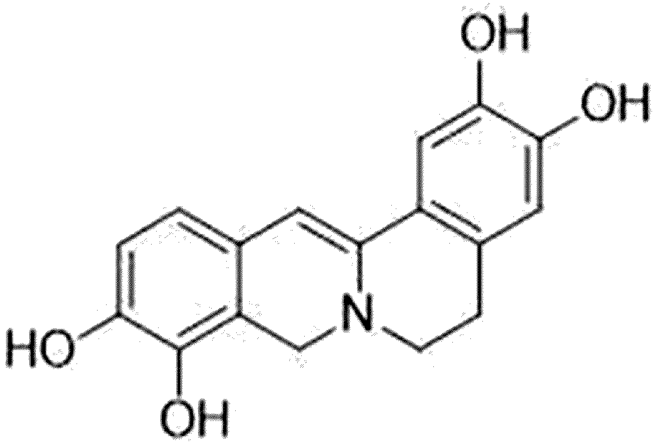

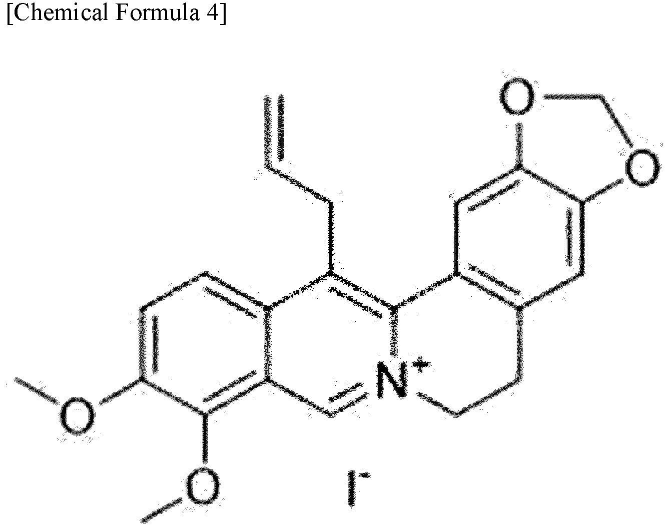

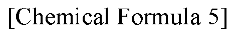

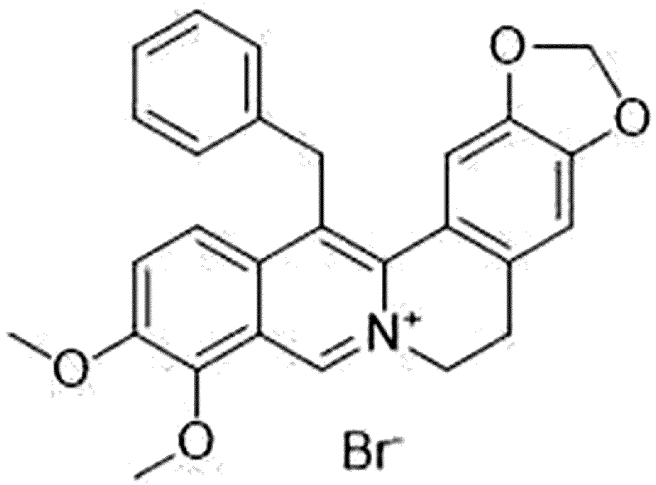

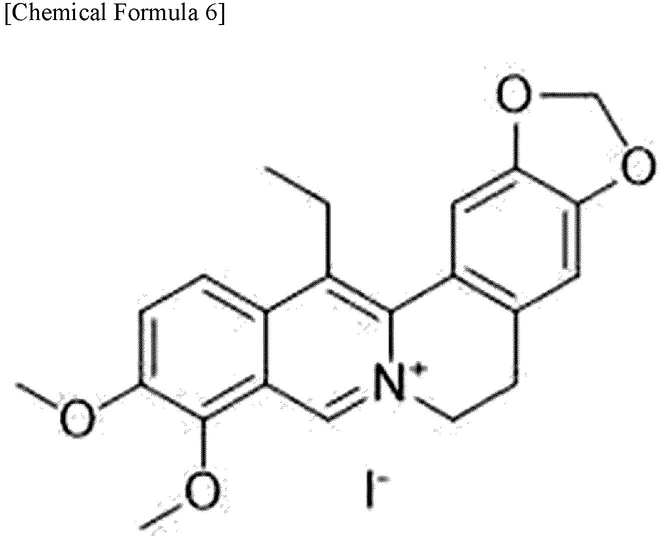

17. A method for increasing mitophagy activity in cells, comprising the step of treating cells with an isoquinoline derivative compound.

18. The method of claim 17, wherein the isoquinoline derivative compound is represented by the following Chemical Formula 1 to Chemical Formula 6.

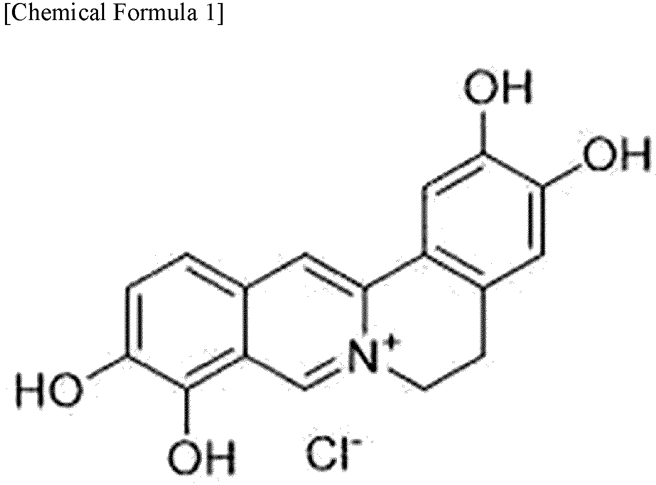

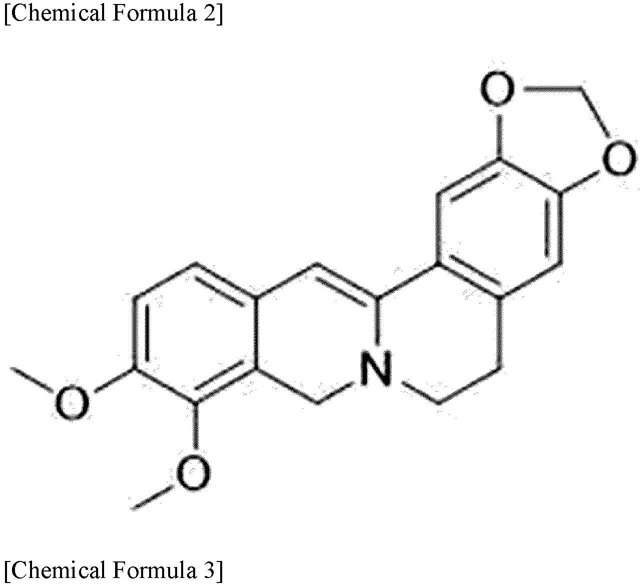

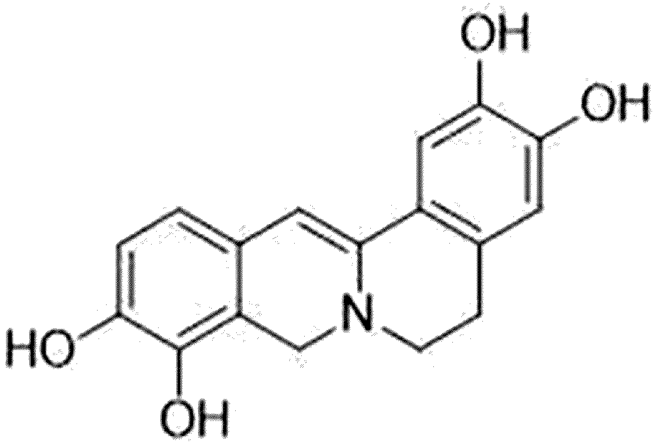

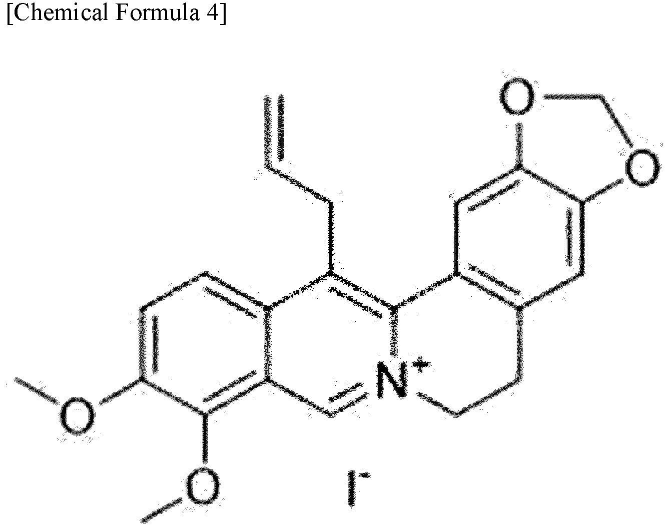

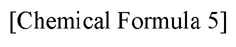

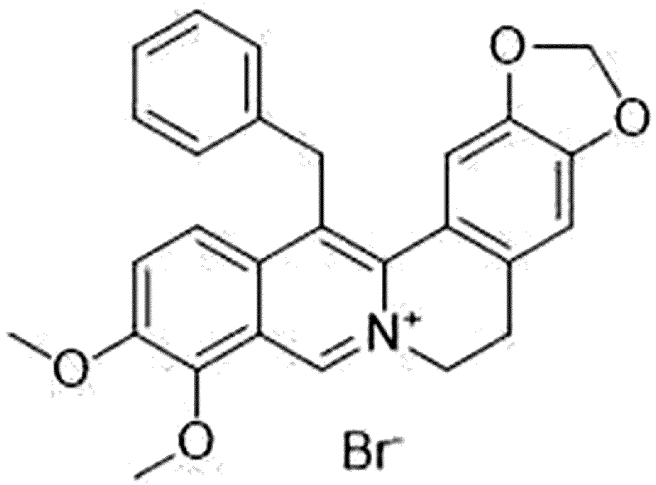

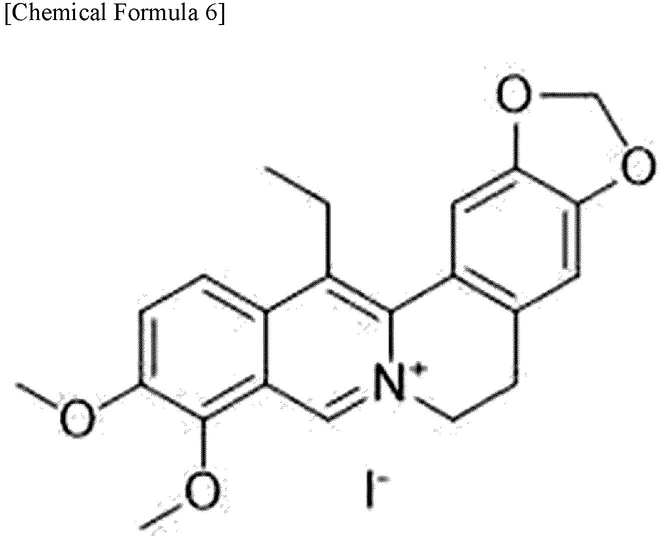

19. A method for inhibiting mitochondrial dysfunction, comprising the step of treating cells with an isoquinoline derivative compound.

20. The method of claim 19, wherein the isoquinoline derivative compound is represented by the following Chemical Formula 1 to Chemical Formula 6.

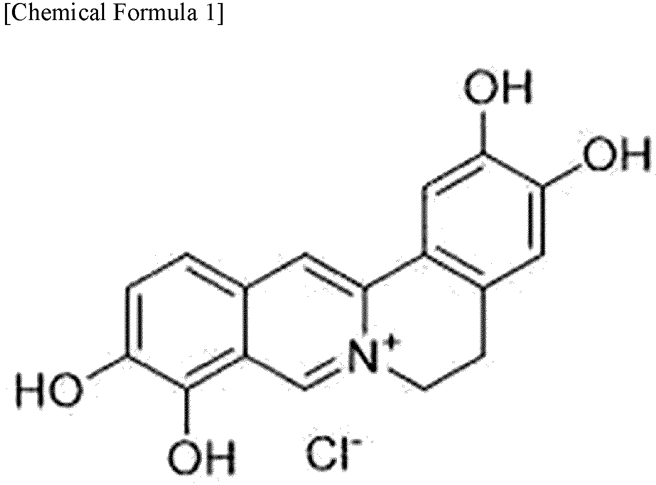

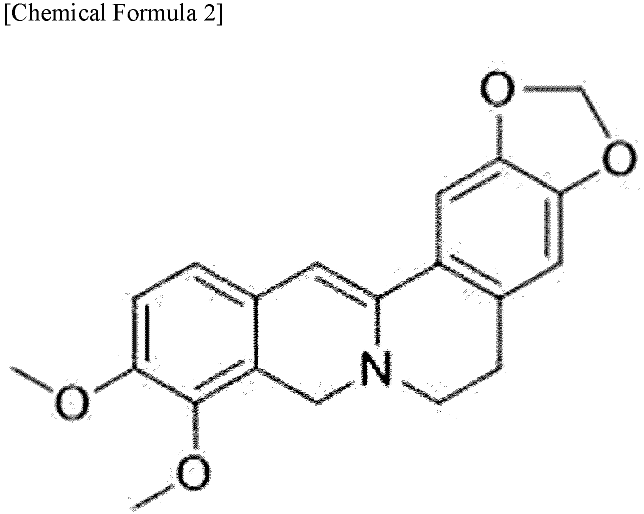

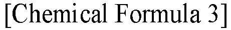

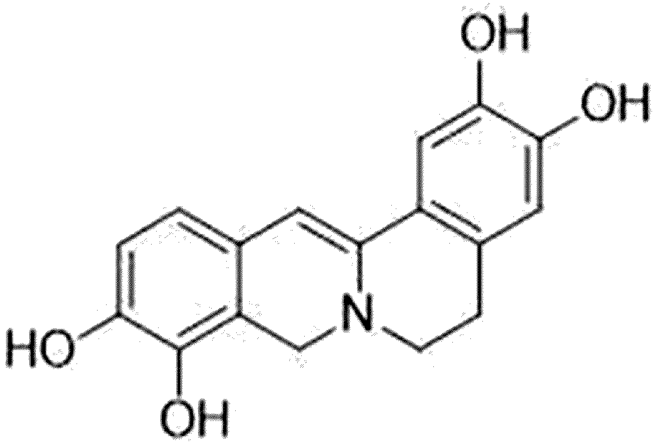

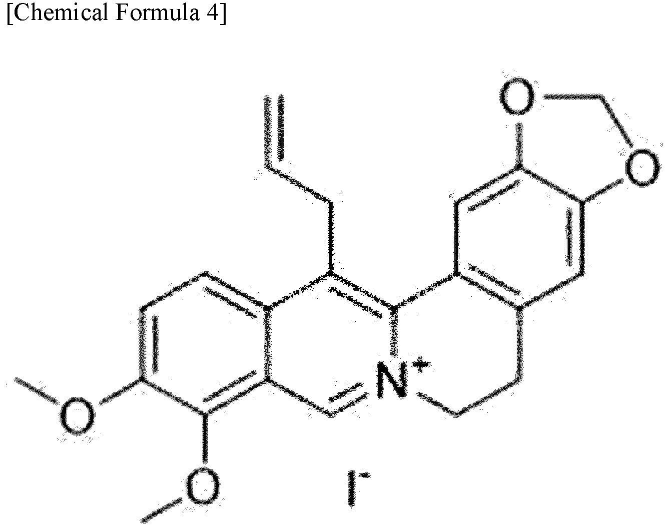

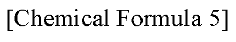

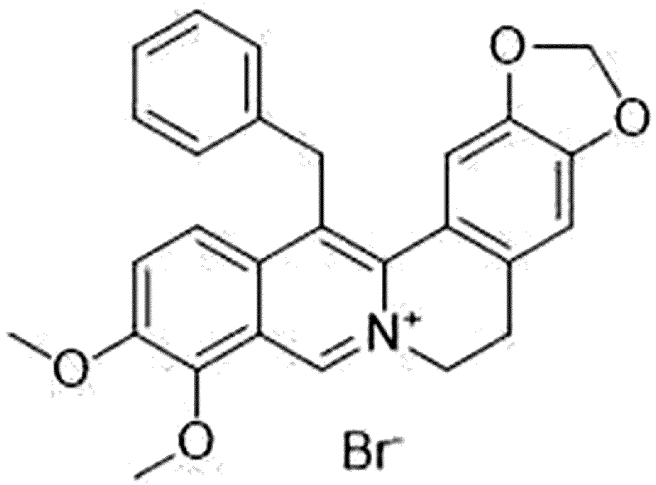

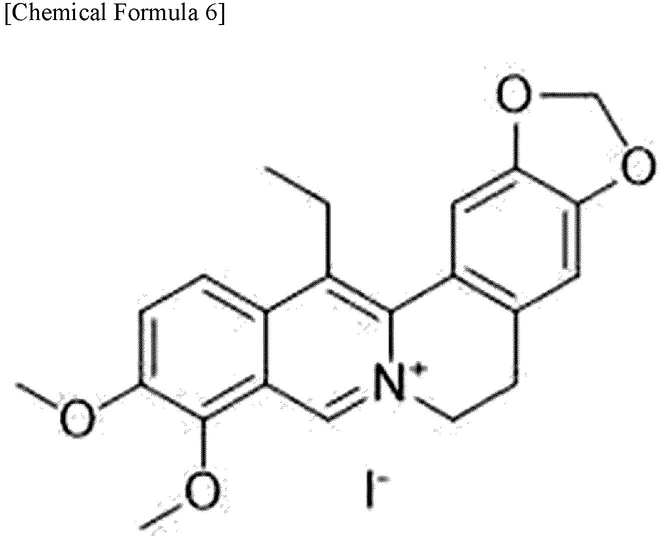

21. A method for the prevention or treatment of disease caused by mitochondrial dysfunction, comprising the step of administering an isoquinoline derivative or a salt thereof to an individual in need thereof.

22. A use of an isoquinoline derivative or a salt thereof for the preparation of a medicament for the prevention or treatment of disease caused by mitochondrial dysfunction.

23. A use of an isoquinoline derivative or a salt thereof for the prevention or treatment of disease caused by mitochondrial dysfunction.
